# EUROPEAN PATENT APPLICATION

(11) **EP 3 726 212 A1**
(43) Date of publication of application: **21.10.2020**
(21) Application number: 19170517.7
(22) Date of filing: 23.04.2019
(51) Int. Cl.: G01N 33/02, A47J 36/32, G09B 19/00

(54) **SYTEM FOR SENSING CHARACTERISTICS OF A FOOD MATERIAL**

(30) Priority: 18.04.2019 PT 2019115466
(71) Applicant: Modelo Continente Hipermercados, S.A., 4464-503 Senhora da Hora (PT)
(72) Inventor: FARIA ROCHA, DUARTE NUNO, 3800-423 Aveiro (PT)
(74) Representative: do Nascimento Gomes, Rui

(57) **Abstract**

The present application is enclosed in the area of kitchen machines, and refers to a system for sensing characteristics of a food material being cooking in the kitchen machine. The system allows monitoring in a continuous basis said food material, so its respective taste can be adjusted during the process according to different cooking profiles, which represents users' taste preferences. The system comprises a sensing module which includes a palatability unit and a scanning unit, that are responsible for monitoring sample parameters collected periodically form the food material and from the cooking procedure. These sample parameters are then compared with reference parameters, generated from the preferences of a user, in order to actuate the kitchen machine operation.

## Description

### TECHNICAL FIELD

The present application is enclosed in the field of kitchen machines, in particular kitchen machines which are electrically run/operated, typically comprising a mixing cup in which automatic mixing actions are performed and operating with automatic cooking programs, and describes a system for sensing characteristics of a food material.

### PRIOR ART

The current kitchen machine apparatus known from the art present the drawback of taste deviation from a desired standard taste which is defined by the preferences of a user. Generally, such devices are operated in accordance with cooking programs that prepare the food according to pre-determined preparation schemes that are stored in said devices in order to be executed. Based on this principle, the document WO2012127412A1 intends to solve the problem of taste deviation by controlling dynamically the preparation scheme in all the different stages of a cooking process, comparing sensor values related to the taste during different stages of the food preparation with the corresponding reference values.

The use of such a scheme based on the comparison between collected data related to taste and a set of reference data is a strategy also followed by documents EP1764708A1 and EP3332678A1. However, as in the case of the solution described in the present application, none of the kitchen machines known in the art provides a complex system able to adapt automatically the taste sensory parameters of a cooking program to a user's taste preferences, in order to create a dynamic reference record, to be compared with the sensor values collected during the cooking process.

Furthermore, the prior art devices do not consider the possibility of different users operating the kitchen machine, each with its specific taste preferences, whereby a dynamic scheme of operation and monitoring of the cooking process is not yet known.

The present solution innovatively solves such issues.

### SUMMARY

The present application relates to a system for sensing characteristics of a food material, suitable for a kitchen machine. In the context of the present application a kitchen machine is to be understood as an electrically ran device and comprising i) a mixing cup inside which ingredients are placed, ii) mixing means provided in the mixing cup, iii) a base station comprising heating and cooling means, the base station suitable for coupling of the mixing cup and which thereby is able to provide motion to the mixing means, iv) means for automatically measuring weight or volume of ingredients placed into the mixing cup, v) a processing unit comprising processing means programmed to execute an automatic cooking program selected from a list of automatic cooking programs stored in a memory module of such processing unit and vi) an interface unit including an input and an output module.

Therefore, it is an object of the present application to describe a system adapted to monitor food materials and the cooking procedure being executed in the kitchen machine, so its respective taste and physical parameters can be adjusted during the cooking process according to different cooking profiles that represent users' preferences. Said preferences are defined by user cooking profile sensory pattern records that are comprised by at least on user palatability parameter which relates to at least one food property. On the other hand, the physical parameters may relate to the volume of the ingredients or may relate to the cooking procedure and its temperature gradients, pressure of levels of humidity.

According to one aspect of the present application, the system is comprised by at least on kitchen machine including the elements i) to vi) as described above, in which each of the automatic cooking programs stored in the memory module comprises:
a set of specific operative instructions configured to actuate the kitchen machine elements i) to iv),
a list of ingredients and respective dosages to be placed inside the mixing cup in order to cook a food material according to the set of specific operative instructions defined in the selected automatic cooking program, and
a standard program sensory pattern record comprised by at least one standard program palatability parameter related to a food property such as salty level, acidity level, sweetness level, bitterness level and/or level of acrid, and by at least one standard program physical parameter that may be related to the volume of each of the referred ingredients, the temperature, pressure and/or humidity levels during the cooking process.

According to another aspect of the present application, the system for sensing characteristics of a food material now developed is comprised by a sensing module which is formed by a palatability unit and by a scanning unit.

The palatability unit is responsible for determining taste deviation of the food material being cooked in relation to an ideal palatability record. It is comprised by a sensory probe, a palatability pattern module comprising a database structure and a correlation engine, and by a comparison module. The sensory probe is connected to the mixing cup and is configured to acquire at least one palatability parameter from the food material being cooked. The database structure of the palatability pattern module is adapted to store at least one user cooking profile sensory pattern record comprised by at least one user palatability parameter, and also to store the standard program palatability parameters of the standard program sensory pattern records of each automatic cooking program stored in the memory module of the kitchen machine's processing unit. The correlation engine of the palatability pattern module is programmed to correlate the at least one standard program palatability parameter of the selected automatic cooking program with the at least one user palatability parameter of the selected user cooking profile sensory pattern record, in order to generate a representative palatability pattern record comprised by at least one representative palatability parameter. The comparison module is programmed to compare the at least one palatability parameter collected by the sensory probe with the at least one representative palatability parameter of the representative palatability pattern record in order to determine taste deviation, which may trigger a feedback action embodied by the output module of the kitchen machine to inform the user of such taste deviation.

The scanning unit of the sensing module is also connected to the mixing cup and comprises a shape detection module, a sensory probe and a comparison module. The shape detection module is configured to determine the volume of the ingredients placed inside the mixing cup. The sensory probe comprises a set of sensors adapted to detect physical parameters associated to the cooking process, such as temperature, pressure and humidity levels inside said mixing cup. In its turn, the comparison module is responsible for comparing the at least one physical parameter collected by the sensory probe or determined by the shape detection module with the at least one standard program physical parameter of a standard program sensory pattern record. The output of this comparison is used by the scanning unit to reprogram the operative instructions for the kitchen machine elements i) to iv) in order to adapt the real physical characteristics of the cooking process to the ideal conditions defined by the standard program physical parameters of the automatic cooking program to be selected.

Advantageously, the kitchen machine may further comprise a container which includes a plurality of compartments suitable for storing additional ingredients such as dried or liquid seasonings, and dispensing means wherein said dispensing means and the mixing cup being such that the ingredients contained in the compartments of the container are dispensable in a configurable dose to the inside of the mixing cup under a dispensing command. Said dispensing command is emitted by the palatability unit as a result of the comparison module's procedure, and comprises information related to which compartment is to be opened in order to add, in a proper dosage, the respective ingredient to the food material being cooked.

It is also an object of the present application a method for operating the system previously described. Said method comprises the steps of:
- Programming initial parameters' configuration related to the selection of an automatic cooking program to be executed by a kitchen machine and the selection of a user's cooking profile, define by a user cooking profile sensory pattern record;
- Generation of a representative palatability pattern record, by correlating each standard program palatability parameter of a standard program sensory pattern record of the selected automatic cooking program with the respective user palatability parameter of the user cooking profile sensory pattern record, wherein said representative palatability pattern record is comprised by at least one representative palatability parameter;
- Detection of at least one palatability parameter, related to at least one food property, from a food material being cooked inside of the mixing cup of the kitchen machine;
- Comparison between the at least one palatability parameter of the food material being cooked and the at least one representative palatability parameter of the representative palatability pattern record generated;
- Detection of physical parameters related to the food material and the cooking procedure, such as volume of ingredients, temperature, pressure and/or humidity;
- Comparison between the detected physical parameters with the respective standard program physical parameters of the standard program sensory pattern record of the selected automatic cooking program;
- Actuation on the kitchen machine operation by providing user feedback information for adjustment of the at least one food property related to the sensory parameters compared and/or by emitting a dispensing command to a container comprised by a plurality of compartments suitable to store ingredients, such as dried or liquid seasonings; said dispensing command comprising information related to the compartment to be opened and to a dose of the ingredient to be dispensed into the mixing cup;
- actuation on the kitchen machine operation by reprogramming the operative instruction of the elements i) and iv).

Such method provides a more accurate cooking preparation scheme, according to users' specific preferences, defined by different cooking profiles, by continuously monitoring the taste of the food material being cooked in the mixing cup and also the cooking procedure being executed by the kitchen machine. Preferably, the actuation step may relate with provide a feedback to the user in order to inform him of the need to adjust the taste of the food material being cooked, or may involve an automatic and dynamic taste adjustment by means of a container with dispensing means wherein additional ingredients are stored.

### DESCRIPTION OF FIGURES

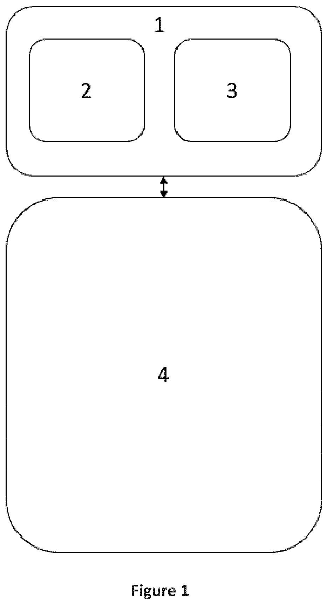

Figure 1 - representation of an embodiment of the system for sensing characteristics of a food material developed, wherein the reference signs represent:
   1 - sensing module;
   2 - palatability unit;
   3 - scanning unit;
   4 - kitchen machine.

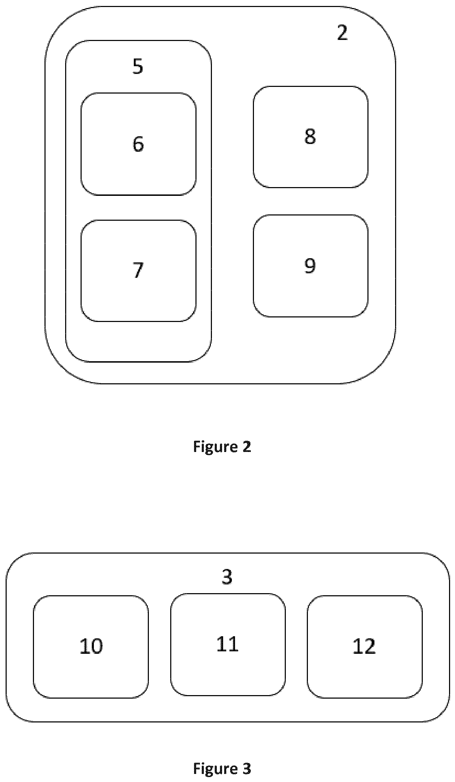

Figure 2 - representation of an embodiment of the palatability unit, wherein the reference signs represent:
   2 - palatability unit;
   5 - palatability pattern module;
   6 - database structure;
   7 - correlation engine;
   8 - sensory probe;
   9-comparison module.
Figure 3 - representation of an embodiment of the scanning unit, wherein the reference signs represent:
   3 - scanning unit;
   10 - shape detection module;
   11 - sensory probe;
   12 -comparison module.

### DETAILED DESCRIPTION

The more general and advantageous configurations of the present application are described in the Summary. Such configurations are detailed below in accordance with other advantageous and/or preferred embodiments of implementation of the technology now developed.

In an inventive aspect of the system of the present application, each automatic cooking program is comprised by supplementary information in addition to the operative instructions defining the cooking procedure used by a kitchen machine. Said supplementary information are related to a list of ingredients and respective dosages to be placed inside the mixing cup, in order to cook a food material according to the referred operative instructions, and to a standard program sensory pattern record used to define specific food properties related to the automatic cooking program. Said food properties are defined by palatability parameters and can be related to salty, acidity, sweetness, bitterness and/or level of acrid, for now on referred as standard program palatability parameters, and also by physical parameters referring to the volume of each ingredient. The cooking procedure can be essentially defined by its temperature gradients, pressure and humidity levels. The number of parameters, of both types, used to characterize a standard program sensory pattern record may vary, but at least such record is comprised by one standard program palatability parameter which is configured by an external entity according to regular taste patterns of a reference user, and by one standard program physical parameter referring to a volume of an ingredient or to a temperature, pressure or humidity levels of the cooking procedure. In the context of the present application a food material is to be understood as the result of mixing and preparation of the ingredients placed in the mixing cup according to the cooking procedure defined by an automatic cooking program. In this context, a dosage is a measured amount of an ingredient in a dried - solid - or liquid state.

In order to sense the characteristics of a food material, in a further inventive aspect of the system now developed, it is comprised by a sensing module. Said sensing module is formed by a palatability unit and by a scanning unit.

In an innovative aspect of the system, the palatability unit is configured to detect taste deviation, and comprises a sensory probe, a palatability pattern module and a comparison module.

The sensory probe is installed, preferably, inside the mixing cup in such a way to be in contact with the food material being cooked, in order to be able to periodically collect food properties related to salty, acidity, sweetness, bitterness and/or level of acrid. For that, and depending on its configuration, the sensory probe is programmed to acquire at least one palatability parameter from food material being cooked. In one embodiment, the sensory probe is comprised by a set of sensors including a taste sensor and/or a tactile sensor used to further determine the creaminess level of the food material. In another embodiment the sensory probe is adapted to detect near infrared spectrum of the food material being cooked. For this purpose, the palatability unit is further comprised by a near infrared spectroscopy engine adapted to determine periodically at least one food property of the food material being cooked, based on a superposition of the infrared spectrum detected by the sensory probe with individual near infrared spectra of each ingredient of the selected automatic cooking program's list of ingredients. The duty cycle of the sensory probe may vary according to the automatic cooking program to be executed in the kitchen machine.

The palatability pattern module of the palatability unit comprises a database structure and a correlation engine. The database structure is adapted to store a) at least one user cooking profile sensory pattern record, comprised by at least one user palatability parameter, and b) the palatability parameters of the standard program sensory pattern records of each automatic cooking program stored in the memory module of the kitchen machine's processing unit. A user cooking profile sensory pattern record can be defined by a user according to its unique taste preferences in relation to food properties such as salty, acidity, sweetness, bitterness and/or level of acrid. The number of palatability parameters used to characterize a user cooking profile may vary according to user's configuration. Alternatively, the user may select a user cooking profile from a list of standard cooking profiles store in the database that characterize regular taste preferences associated to a reference user. In one embodiment, the user cooking profile sensory pattern record is configured by the user using the input module of the kitchen machine's interface unit. In one embodiment such input module is a keyboard of a software type, wherein said keyboard is touch-based and comprises a liquid crystal display screen where an array of virtual keys is displayed to permit the user to perform an input operation by touching the software keyboard. In another embodiment the keyboard is of an hardware type, comprising a set of real keys to be pressed by an user to perform an input operation. In another embodiment, the configuration of the user cooking profile sensory pattern record is perform using an external processing unit, and for such, the interface unit further comprises a wireless communication module and a user interface platform adapted to provide user access to said platform through the wireless communication module, by means of an application interface running in said processing device, which is configured to communicate with the user interface platform by means of a wide area network, local area network or personal area network. In a preferred embodiment, the external processing device is a mobile device.

The correlation engine of the palatability pattern module is operatively connected to the database structure, and is programmed to correlate the standard program palatability parameters of the standard program sensory pattern record of the selected automatic cooking program with the user palatability parameters of the user cooking profile sensory pattern record of the selected user's cooking profile, in order to generate a representative palatability pattern record comprised by at least one representative palatability parameter. The correlation operation performed by such engine involves equalizing each of the at least one standard program palatability parameter of the standard program sensory pattern record with the respective user palatability parameter of the user cooking profile sensory pattern record. Particularly, a representative palatability parameter of a representative palatability pattern record is generated by equalizing the standard program palatability parameter of the standard program sensory pattern record, related to a specific food property, with the respective user palatability parameter, related to the same specific food property, of the user cooking profile sensory pattern record.

The comparison module of the palatability unit is a processing engine operatively coupled to the palatability pattern module, being programmed to compare the at least one palatability parameter collected by the sensory probe with the at least one representative palatability parameter of the representative palatability pattern record generated by the correlation engine. Particularly, the comparison module is operable to compare the at least one palatability parameter collected by the sensory probe and related to a specific food property with the respective representative palatability parameter related to the same specific food property. This comparison allows to determine taste deviation and based on that, the comparison module is able to determine the required amount of a certain ingredient in order to provide user feedback information of adjustment of the food properties related to the palatability parameters compared, by being operatively coupled to the output module of the interface unit. Said output module is comprised by output means, preferably a liquid crystal display and an audio speaker. In one embodiment, the userfeedback information is a visual information to be displayed in the liquid crystal display screen and/or an audio information to be emitted by the audio speaker.

In an innovative aspect of the system, the scanning unit of the sensing module is configured to detect the physical characteristics of the food material and also of the cooking procedure, and is comprised by a shape detection module, a sensory probe, a comparison module.

The shape detection module is configured to determine the volume of the ingredients placed inside the mixing cup. To achieve that purpose, this module comprises a 3D imaging system that may be based on many different technologies already known from the art, in particular any of contact or non-contact types of scanning technologies. In one embodiment, the 3D imaging system is of a contact type, comprising a contact element that probes the ingredients' body through physical touch. in another embodiment, the 3D imaging system is of a non-contact type, comprising an emitter to emit a radiation or light and a receiver to detects its reflection or radiation passing through an ingredient's body. Possible types of emissions used may include light, ultrasound or x-ray.

The sensory probe of the scanning unit is installed inside the mixing cup, in order to be able to periodically collect data related to physical parameters associated to the cooking procedure being executed. For that, the sensory probe is programmed to acquire at least one physical parameter related to temperature, pressure or humidity levels.

The comparison module of the scanning unit comprises processing means adapted to compare the physical parameters collected from the sensory probe and from the shape detection module of the scanning unit with the standard program physical parameters of the standard program sensory pattern record associated to the selected automatic cooking program. Particularly, the comparison module is operable to compare the at least one physical parameter collected by the sensory probe and/or determined by shape detection unit of the scanning unit, related to a specific physical property of the food material or of the cooking procedure, with the respective standard program physical parameter, related to the same specific physical property, of the standard program sensory pattern record assigned to the selected automatic cooking program.

In an advantageous embodiment, the scanning unit of the sensing module is able to reprogram the operative instructions of the selected automatic cooking program. In fact, by having the physical parameters of the cooking procedure monitored it is possible for the scanning unit to reprogram the operative instructions of the selected automatic cooking program, in order to adapt the real physical characteristics of the cooking process to the ideal conditions defined by the standard program physical parameters associated to automatic cooking program to be selected. The reprograming of the operative instructions implies changing the operative working conditions of the elements i) to iv) of the kitchen machine, in order to match those defined by default - according to the selected automatic cooking program - and may involve configuration of rotational speeds, power levels, heating/cooling time frames and setting values, among others.

In an advantageous embodiment, the kitchen machine of the system is further comprised by a container including a plurality of compartments, each compartment having an identification code, and suitable for storing ingredients, particularly, dried or liquid seasonings. The identification code may be a symbol or a numerical reference and is used to unequivocally identify each compartment of the container and the respective ingredient therein stored, so the automatic adjustment of taste can be performed correctly. Said container also comprises dispensing means, wherein said dispensing means and the mixing cup of the kitchen machine being such that the ingredients contained in the compartments of the container are dispensable in a configurable dose to the inside of the mixing cup under a dispensing command. Said dispensing command is emitted by the palatability unit, as a result of the comparison module's procedure, and comprises information related to the identification code of at least one compartment containing the required ingredient to perform taste adjustment and the respective dosage information. The configuration of each dose is determined by the comparison module of the palatability unit. In one embodiment, the dispensing means of the container may comprise a controllable gate operatively connected to the means for automatically measuring weight or volume of ingredients of the mixing cup to allow a more accurate dispensing procedure of an ingredient according to the specific dose determined by the comparison module.

The present application also relates to a method for operating the system for sensing characteristics of a food material described. In an inventive aspect of the method, it involves an initial step of configuration where a user may select in addition to the automatic cooking program to be executed by the kitchen machine, a user's cooking profile to be used. It will be based on those parameters that the taste deviation will be determined and a taste adjustment will be performed. The next step involves the generation of a representative palatability pattern record, comprised by at least one representative palatability parameter, by correlating each standard program palatability parameter of the standard program sensory pattern record associated to the selected automatic cooking program running in the kitchen machine, with the respective user palatability parameter of the user cooking profile sensory pattern record assigned to the selected user's cooking profile. The correlation step is performed by equalizing the standard program palatability parameter of the standard program sensory pattern record, related to a specific food property, with the respective user palatability parameter, related to the same specific food property, of the user cooking profile sensory pattern record. Then, a detection step is executed by means of a sensory probe installed in a mixing cup of the kitchen machine, which will collect at least one palatability parameter, related to at least on food property, from the food material being cooked inside of said mixing cup. The next step involves the use of a comparison module to perform a comparison between the at least one palatability parameter collected by the sensory probe, related to a specific food property, with the at least one representative palatability parameter, related to the same specific food property, of the representative palatability pattern record generated by the correlation engine. The next step involves the detection of physical parameters of the food material being cooked and of the cooking process being executed, such the volume of the ingredients, and the temperature gradients, pressure or humidity levels sensed inside the mixing cup. Then, a comparison is performed between the detected physical parameters and the respective standard program physical parameters of the standard program sensory pattern record associated to the selected automatic cooking program. Said comparison implies comparing the at least one physical parameter collected by the sensory probe and/or determined by shape detection unit of the scanning unit, related to a specific physical property of the food material or of the cooking procedure, with the respective standard program physical parameter, related to the same specific physical property, of the standard program sensory pattern record assigned to the selected automatic cooking program.

Once the physical and the palatability parameters are accessed, an actuation on the kitchen machine is provoked which may involve, in one embodiment, reprogramming the operative instructions of the elements i) to iv) of the kitchen machine, such as configuration of rotational speeds, power levels or heating/cooling time frames. In another embodiment, the actuation on the kitchen machine may imply providing user feedback information for adjustment of the at least one food property related to the palatability parameters compared in the palatability unit. Additionally, in another embodiment, the palatability unit may be programmed to issue, upon execution of its comparison unit's procedure, a dispensing command to a container comprised by a plurality of compartments suitable to store dried or liquid seasoning. Said dispensing command comprises information related to the compartment to be opened and the configurable dose of the dried or liquid seasoning to be dispensed into the mixing cup.

In an advantageous embodiment, the method further includes an initial stage of calibration, to be executed prior to the step of configuration of the cooking profile and the automatic cooking program. Said calibration stage is used by the user to configure at least one user palatability parameter, related to at least one food property, of the user cooking profile sensory pattern record assign to a user's cooking profile.

In another embodiment, certain steps of the method are executed cyclically during the execution of the automatic cooking program in the kitchen machine. In particular, the stages of a) detection of palatability parameters and physical parameters inside of the mixing cup of a kitchen machine during the cooking procedure, b) comparison of said palatability and physical parameters with the respective reference records and c) actuation on the kitchen machine operation, may be performed periodically during the execution of the selected automatic cooking program.

As will be clear to one skilled in the art, the present application should not be limited to the embodiments described herein, and a number of changes are possible which remain within the scope of the present application.

Of course, the preferred embodiments shown above are combinable, in the different possible forms, being herein avoided the repetition all such combinations.

## Claims

1. System for sensing characteristics of a food material, suitable for a kitchen machine, such kitchen machine being electrically run and comprising i) a mixing cup inside which ingredients are placed, ii) mixing means provided in the mixing cup, iii) a base station comprising heating and cooling means, said base station suitable for coupling of the mixing cup and which thereby is able to provide motion to the mixing means, iv) means for automatically measuring weight or volume of ingredients placed into the mixing cup, v) a processing unit comprising processing means programmed to execute an automatic cooking program selected from a list of automatic cooking programs stored in a memory module of such processing unit and vi) an interface unit including an input and an output module; the system being **characterized in that** it comprises at least one kitchen machine including the elements i) to vi) as described, in which each of the automatic cooking programs stored in the memory module comprises:
- a set of specific operative instructions configured to actuate the kitchen machine elements i) to iv);
- a list of ingredients and respective dosages, to be placed inside the mixing cup, in order to cook a food material according to a cooking procedure defined by the set of specific operative instructions;
- a standard program sensory pattern record comprised by at least one standard program palatability parameter related to a food property and by at least one standard program physical parameter related to the ingredients and the cooking procedure;
the system further comprises a sensing module comprising a palatability unit and a scanning unit, wherein
The palatability unit comprises:
- a sensory probe connected to the mixing cup, configured to acquire at least one palatability parameter from the food material being cooked;
- a palatability pattern module comprising:
a database structure adapted to store a) at least one user cooking profile sensory pattern record, related to a user's cooking profile to be selected, comprised by at least one user palatability parameter and to store b) the standard program palatability parameters of the standard program sensory pattern records of each automatic cooking program stored in the memory module of the kitchen machine's processing unit;
a correlation engine programmed to correlate the standard program palatability parameters of the standard program sensory pattern record of the automatic cooking program to be selected with the user cooking profile sensory pattern record, in order to generate a representative palatability pattern record comprised by at least one representative palatability parameter;
- a comparison module programmed to compare the at least one palatability parameter collected by the sensory probe of the palatability unit with the at least one representative palatability parameter of the representative palatability pattern record; and
the scanning unit comprises:
- A shape detection module, connected to the mixing cup, comprising a 3D imaging system configured to determine the volume of the ingredients placed inside the mixing cup;
- A sensory probe, connected to the mixing cup, adapted to collect at least one physical parameter;
- A comparison module programmed to compare the at least one physical parameter collected from the sensory probe and/or determine by the shape detection module of the scanning unit with the at least one standard program physical parameter assigned to automatic cooking program to be selected.

2. System according to claim 1, wherein the standard program palatability parameter and the palatability parameter are related to one of the following food properties: salty level, acidity level, sweetness level, bitterness level and/or level of acrid; and the standard program physical parameter and the physical parameter are related to one of the following: volume of the ingredients, temperature, pressure and humidity levels of the cooking procedure.

3. System according to any of the previous claims, wherein the sensory probe of the palatability unit comprises a set of sensors including a taste sensor.

4. System according to claim 3, wherein the set of sensors of the sensory probe further includes a tactile sensor, and the sensory parameter acquired by the sensory probe further relates to creaminess level.

5. System according to claim 2, wherein:
- the sensory probe of the palatability unit is adapted to detect near infrared spectrum of the food material to be cooked; and
- the palatability unit further comprises a near infrared spectroscopy engine adapted to determine at least one food property of the food material to be cooked, based on a superposition of the infrared spectrum detected by the sensory probe with individual near infrared spectra of each ingredient of the selected automatic cooking program's list of ingredients.

6. System according to any of the previous claims, wherein the user palatability parameter associated to a user cooking profile sensory pattern record is configured by a user:
- by means of the input module of the interface unit; said input module of a keyboard type; and/or
- using a processing device, preferably a mobile device, wherein the interface unit further comprises a wireless communication module and a user interface platform adapted to provide user access to said platform through the wireless communication module, by means of an application interface running in said processing device, which is configured to communicate with the user interface platform by means of a wide area network, local area network or personal area network.

7. System according to any of the previous claims, wherein the correlation engine of the palatability unit is operable to:
- Generate at least one representative palatability parameter by equalizing each of the at least one standard program palatability parameter of the standard program sensory pattern record associated to the automatic cooking program to be selected with the respective user palatability parameter of the user cooking profile sensory pattern record;
- Generate a representative palatability pattern record, comprised by the at least one representative palatability parameter.

8. System according to any of the previous claims, wherein the comparison module of the palatability unit is operable to:
- compare the at least one palatability parameter collected by the sensory probe of the palatability unit with the respective representative palatability parameter;
- provide user feedback information of adjustment of the food properties related to the palatability parameters compared, by being operatively coupled to the output module of the interface unit; said output module comprised by output means, preferably a liquid crystal display and an audio speaker, wherein the user feedback information is a visual information to be displayed in the liquid crystal display screen and/or an audio information to be emitted by the audio speaker.

9. System according to any of the previous claims, wherein the kitchen machine further comprises a container, said container comprising:
- a plurality of compartments, each compartment having an identification code, and suitable to store ingredients, particularly, dried or liquid seasonings;
- dispensing means, wherein said dispensing means and the mixing cup being such that the ingredients contained in the compartments of the container are dispensable in a configurable dose to the inside of the mixing cup under a dispensing command; said dispensing command is emitted by the comparison module of the palatability unit and comprises information related to the identification code of the compartment to be opened and the configurable dose of the ingredient therein stored to be dispensed into the mixing cup.

10. System according to any of the previous claims, wherein comparison module of the scanning unit is operable to compare the at least one physical parameter collected by the sensory probe of the scanning unit with the respective standard program physical parameter associated to the automatic cooking program to be selected.

11. System according to claim 10, wherein the scanning unit is operable to reprogram the operative instructions the automatic cooking program to be selected based on the output of the comparison module of the scanning unit, by configuring operative working conditions of the elements i) to iv) of the kitchen machine related to rotational speeds, power levels and/or heating/cooling time frames and setting values.

12. Method for operating the system for sensing characteristics of a food material of claims 1 to 11, comprising the following steps:
- Selection of an automatic cooking program to be executed by a kitchen machine;
- Selection of a user's cooking profile;
- Generation, by means of the correlation engine of the palatability unit, of a representative palatability pattern record, by correlating each standard program palatability parameter of the standard program sensory pattern record of the selected automatic cooking program with the respective user palatability parameter of the user cooking profile sensory pattern record of the selected user's cooking profile; said representative palatability pattern record being comprised by at least one representative palatability parameter;
- Detection, by means of the sensory probe of the palatability unit, of at least one palatability parameter, related to at least one food property, from a food material being cooked inside of the mixing cup of a kitchen machine;
- comparison, by means of the comparison module of the palatability unit, of the at least one palatability parameter collected by the sensory probe, related to a specific food property with the at least one representative palatability parameter, related to the same specific food property, of the representative palatability pattern record generated by the correlation engine of the palatability unit;
- Detection, by means of the scanning unit, of physical parameters of the food material and of the cooking procedure;
- Comparison, by the comparison module of the scanning unit, between the detected physical parameters with the respective standard program physical parameters of the standard program sensory pattern record of the selected automatic cooking program;
- actuation on the kitchen machine operation.

13. Method according to claim 12, further comprising an initial stage of calibration, wherein the at least one user palatability parameter of the user cooking profile sensory pattern record is configured by a user; and wherein
the actuation step includes:
- providing user feedback information for adjustment of the at least one food property related to the sensory parameters compared; and/or
- emitting a dispensing command, by the comparison module, to a container comprised by a plurality of compartments suitable to store ingredients such as dried or liquid seasonings; said dispensing command comprising information related to the compartment to be opened and the configurable dose of the respective ingredient to be dispensed into the mixing cup; and/or
- reprograming the operative instructions of the selected automatic cooking program, by the scanning unit, by configuring operative working conditions of the elements i) to iv) of the kitchen machine related to rotational speeds, power levels and/or heating/cooling time frames and setting values.

14. Method according to any of the previous claims 12 to 13, wherein
the representative palatability parameter is generated by equalizing the standard program palatability parameter of the standard program sensory pattern record, related to a specific food property, with the respective user palatability parameter, related to the same specific food property, of the user cooking profile sensory pattern record of the selected user's cooking profile; and wherein
the comparison, in the comparison module of the scanning unit, implies comparing the at least one physical parameter collected by the sensory probe and/or determined by shape detection unit of the scanning unit, related to a specific physical property of the food material or of the cooking procedure, with the respective standard program physical parameter, related to the same specific physical property, of the standard program sensory pattern record assigned to the selected automatic cooking program.

15. Method according to any of the previous claims 12 to 14, wherein the steps of:
- detection of palatability and physical parameters by the palatability unit and by the scanning unit;
- comparison of said palatability and physical parameters in the comparison modules of the respective palatability unit and scanning unit;
- actuation on the kitchen machine operation,
are performed periodically, during the execution of the selected automatic cooking program.
